# EUROPEAN PATENT SPECIFICATION

(11) **EP 0 873 103 B1**
(45) Date of publication and mention of the grant of the patent: **03.04.2002**
(21) Application number: 96943609.6
(22) Date of filing: 06.12.1996
(51) Int. Cl.: A61F 13/56, A61F 13/15

(54) **INDEPENDENT GARMENT SOILING PROTECTIVE DEVICE FOR USE BETWEEN UNDERGARMENT AND ABSORBENT ARTICLE**
UNABHÄNGIGE VORRICHTUNG ZUM SCHUTZ GEGEN VERSCHMUTZUNG VON KLEIDUNG ZUR BENUTZUNG ZWISCHEN UNTERBEKLEIDUNG UND ABSORBIERENDEM ARTIKEL
DISPOSITIF INDEPENDANT DE PROTECTION ANTI-SOUILLURE DES VETEMENTS A UTILISER ENTRE UN SOUS-VETEMENT ET UN ARTICLE ABSORBANT

(30) Priority: 14.12.1995 US 572650
(43) Date of publication of application: 28.10.1998
(73) Proprietor: THE PROCTER & GAMBLE COMPANY, Cincinnati, Ohio 45202 (US)
(72) Inventor: ALDRICH, Ronald, J., Cincinnati, OH 45251 (US)
(74) Representative: Hirsch, Uwe Thomas
(86) International application number: US9619401
(87) International publication number: WO9721411

(56) References cited:
- WO-A-95/08311
- WO-A-97/00655
- US-A- 4 862 574

## Description

The present invention is generally directed to the protection of undergarments for improved hygiene. More particularly, the present invention is directed to a protective device that is fitted between the undergarment of a user and an absorbent article such as a disposable menstrual pad.

### 2. Background Art

Absorbent articles have been used since ancient times to improve hygiene by absorbing human body fluids. Such articles have traditionally included a variety of absorbent devices such as feminine napkins and pads and panty shields and liners. These articles, while varying in shape, size and construction, are all generally directed to the absorption of body fluids. Absorbed body fluids cannot stain undergarments, while wandering fluids can.

However, while conventional pads, napkins, shields and liners are for the most part effective in absorbing these fluids, leakage can and often does occur. Typical leakage occurs to one side or the other of the absorbent article. The problem is particularly acute during the second and third days of the typical four or five day menstrual cycle during which time hemorrhaging is most pronounced.

A variety of approaches have been taken to resolve the problem of leakage. The general approach taken has been to incorporate wing- or flap-like panels that extend from the longitudinal side edges of the central absorptive body. There is a virtual plethora of examples of this construction, although reference should be particularly made to United States Patent No. 4,589,876, issued on May 20, 1986 to Van Tilburg for "Sanitary Napkin" and United States Patent No. 4,687,478 issued on August 18, 1987, also to Van Tilburg for "Shaped Sanitary Napkin With Flaps", both patents being commonly assigned to the assignee of the instant application.

A variant of an absorbent article directed to the prevention of leakage is disclosed in United States Patent No. 5,037,418, issued on August 6, 1991, to Kons and Paul for an "Absorbent Article Having An Attachable Undergarment Protective Sheet". This patent discloses an absorbent article that comprises a longitudinal pad and an optional liquid-impermeable protective sheet for attachment to a pair of adhesive strips secured axially on the sides of the longitudinal pad. The protective sheet wraps around the underside of the undergarment.

The typical consumer would rather use a particular article with which she is most comfortable so that she can accomplish protection against leakage at the same time. Furthermore, and particularly with the excessively complex structure of Kons et al., in mind, the elimination of leaks can only be practically accomplished through a cost-effective and practical design. During "heavy flow" days, it is not realistic to expect the user to undertake the complex removal and installation of the pad of Kons et al., thus rendering it impractical. In addition, the winged construction of many of the "leak proof" designs renders these articles not only expensive, but inconvenient to change, particularly during peak times of menstrual discharge when frequent changing is necessary.

In a further effort to overcome the inadequacies of known protective devices, an undergarment protector in the form of an adhesive attachment was developed and is disclosed in United States Patent No. 4,862,574, issued on September 5, 1989, to Seidy for "Panty Protector". This patent discloses an undergarment protector having adhesive attachment strips and transversely extending flaps. The attachment strips are provided on both the body of the protector and on the transversely extending flaps. The user removes protective release strips from the adhesive elements on both the body of the protector and the flaps. The protector is then positioned into the crotch portion of the undergarment, and the flaps are encircled around the edges of the leg holes of the undergarment. Pressure applied to both the body of the protector and the flaps adheres the protector to the crotch portion of the undergarment.

While overcoming many of the difficulties of leakage present in earlier absorbent articles, the improvements of Seidy are advantageous, but because of the elaborate adhesive scheme, fall short of providing a protection that is inexpensive to manufacture and convenient to use.

It is therefore an object of the present invention to overcome the disadvantages associated with known hygienic feminine pads, napkins, panty shields and liners by providing a protective device for use between an undergarment and an absorbent article.

It is a further object of the present invention to provide such a device that can be easily attached to and removed from the inner side of the crotch area of an undergarment.

Yet another object of the present invention is to provide a device having a surface to which an absorbent pad may be readily attached or removed thereby allowing the user to change the pad without the need to change the device at the same level of frequency.

Still another object of this invention is to provide a protective device having a device-to-undergarment adhesive strength that is greater than the strength of the absorbent article-to-device adhesive strength thereby allowing the user to remove the absorbent article without unintentionally removing the device.

### SUMMARY OF THE INVENTION

The present invention achieves these objectives in an improved protective device, having the features set forth in claim 1, which comprises a body having two opposed and laterally extending side extensions. The device has an upper absorbent article attachment surface and a lower undergarment contacting surface.

The device is multi-layered and preferably is comprised of three layers. The top layer, which comprises the upper absorbent article attachment surface, is preferably comprised of a non-woven material, such as a spunbonded material. The intermediate layer is preferably comprised of webbed construction and provides the device with bulk and loft. The bottom layer, which comprises the lower undergarment contacting surface, is comprised of a polymerized barrier film.

Intermediate of the side extensions and the body of the device are extensible regions that are corrugated. The extensible regions have a greater range of extension than either the side extensions or the body. Once in place in the undergarment, the device naturally takes on an arcuate shape because of its being nested with the arcuately-shaped crotch area of the undergarment. The extensible regions relieve stresses exerted on the side extensions when the side extensions are folded downward relative the wearer's body to follow the shape of the curved sides of the wearer's undergament. The device of the present invention may take on a personalized shape to fit the wearer, and once so shaped, holds the shape through multiple changings of the absorbent article. The personalized fit also allows the device to remain in place without a complex system of adhesive strips.

Other objects and advantages of the present invention will be made apparent as the description progresses.

### BRIEF DESCRIPTION OF THE DRAWINGS

The various advantages of the present invention will become apparent to one skilled in the art by reading the following specification and appended claims and by referencing the following drawings in which:
Figure 1 is a top plan view of a protective device according to the present invention;
Figure 2 is a perspective view of the protective device of the present invention, as it would appear in place in the crotch area of the user's undergarment;
Figure 3 is a transverse cross-sectional view of the protective device of the present invention in place between the absorbent article and the undergarment;
Figure 4 is a transverse cross-sectional view of the protective device of the present invention illustrating its multi-layered construction;
Figure 5 is a partly exploded perspective view showing the manner of placing the protective device of the present invention in an undergarment; and
Figure 6 is a fragmentary coronal view of an individual and the protective device in place as it would be when in use between an absorbent article and an undergarment.

### DETAILED DESCRIPTION OF THE PREFERRED EMBODIMENT

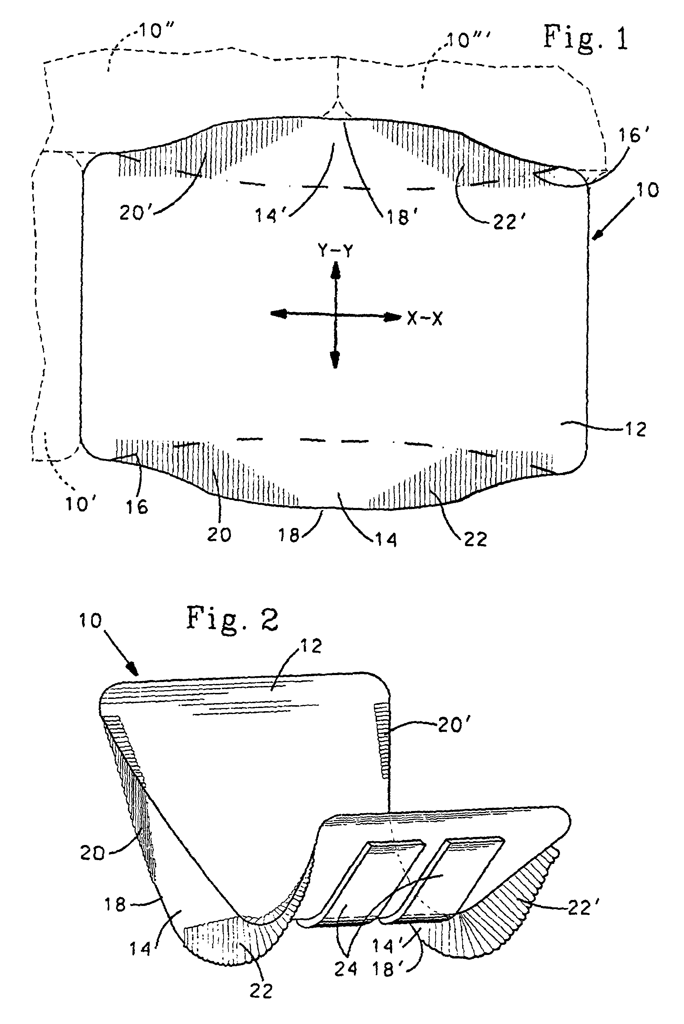

A preferred embodiment of the protective device of the present invention for use between an undergarment and an absorbent article is shown in Figures 1 and 2. With respect first to Figure 1, a plan view of a protective device, generally illustrated as 10, is shown. The protective device 10 comprises an elongated body 12 and a pair of side extensions 14, 14' that extend laterally outwardly from the elongated body 12.

The side extensions 14, 14' are associated with the elongated body 12 along a pair of non-linear lines of juncture 16, 16'. As used in the context of the present specification, the term "non-linear" refers to any various curved lines as opposed to straight lines. In this particular instance, the non-linear lines of juncture 16, 16' generally follow the curved, crotch area edges that define the leg holes of the undergarment. The lines of juncture 16, 16' are shown in broken lines. The lines of juncture 16,16' may or may not be defined on the elongated body 12 by score lines or a pressure-formed seam or perforation. The lines of juncture 16, 16' may also be regarded as lines of flexibility. In any event, it is preferably along these lines of juncture 16, 16' that the side extensions fold when worn, as shown in Figure 2 and as described below.

As used herein, the term "joined" encompasses configurations whereby an element is directly secured to the other element by affixing the element directly to the other element; configurations whereby the element is indirectly secured to the other element by affixing the element to intermediate member(s) which in turn are affixed to the other element; and configurations whereby one element is integral with another element, i.e., one element is essentially part of the other element.

The side extensions 14, 14' have a pair of distal edges 18, 18', respectively, which are remote from the lines of juncture 16, 16'. In the embodiment illustrated in Figure 1, the lines of juncture 16, 16' are concave relative to the distal edges 18, 18'. In this embodiment, the lines of juncture 16, 16' define the plan view shape of the protective device 10 when the user's legs are inserted through the leg holes of an undergarment and the side extensions 14, 14' are folded "down". The shape defined is narrower in its central region than at its ends. Sanitary protective devices that are narrower in the center than at the ends are generally perceived by the user as being more comfortable than those having a uniform width.

The overall dimensions of the protective device 10 may be varied as necessary depending on the size and style of the undergarment and the intended use of the protective device 10. For example, when the user is lying down, there is a tendency for body fluid to gravitate toward either the person's frontside or backside, depending on which side the person is lying. The protective device 10 may accordingly be longer for this purpose. However, with the illustrated X- and Y-axes being oriented by reference to a planar Cartesian coordinate system wherein the longitudinal centerline of the protector, line X-X, is the X-axis and the transverse centerline, line Y-Y, is the Y-axis, the preferred size is between 150 and 170 mm along the X-axis (or the long axis) of the protective device 10 and between 105 and 125 mm along the Y-axis or along the width of the protective device 10.

The protective device 10 is provided with a plurality of extensible regions 20, 20', 22, 22'. The extensible regions 20, 20', 22, 22' allow the side extensions 14, 14' to be folded when worn (as illustrated in Figure 2) without stresses being exerted on the protective device 10 and without the protective device 10 bunching. The extensible regions 20, 20' 22, 22' can be primarily extensible along the X-axis (that is, they are extensible more along the X-axis than along the Y-axis). Alternatively, the extensible regions 20, 20', 22, 22' can be primarily extensible along the Y-axis, or in any direction between the X-axis and the Y-axis. The extensibility of all of the extensible regions 20, 20', 22, 22' can be in the same direction. Alternatively, one or more of the extensible regions 20, 20', 22, 22' can be extensible in a different direction.

The extensible regions 20, 20', 22, 22' may be produced to allow expansion in a variety of ways. However, perhaps the most cost-effective manner of construction is the preferred one, which is to corrugate the extensible regions 20, 20', 22, 22' by forming selected areas through compression. The corrugation is made such that its parallel and alternating ridges and grooves are disposed transverse the long axis of the elongated body 12.

Figure 1 also shows a method of producing the protective device 10. An exemplary series of protective devices 10, 10', 10", 10"' are outlined for cutting from a single sheet of material, with the protective devices 10', 10", 10"' being shown in broken lines. As may be seen, the shape of the protective device 10 is such that many clones of it may be cut from a single sheet with only minimal waste.

With reference specifically to Figure 2, a perspective view of the protective device 10 of the present invention is shown as it would appear in the crotch area of the user's undergarment. The elongated body 12 of the protective device 10 assumes the illustrated arcuate shape when worn by the user because it takes the form of the user's crotch. In addition to assuming the illustrated arcuate shape, the extensible regions 20, 20', 22, 22' allow the side extensions 14, 14' to automatically fold to an acute angle around the side edges of the crotch of the wearer's panties. The corrugations of the extensible regions 20, 20', 22, 22' compensate for the downfolding of the sides 14, 14'.

A plurality of fastening adhesive strips 24 are provided on the underside of the protective device 10 for attachment to an undergarment, as will be described more fully below.

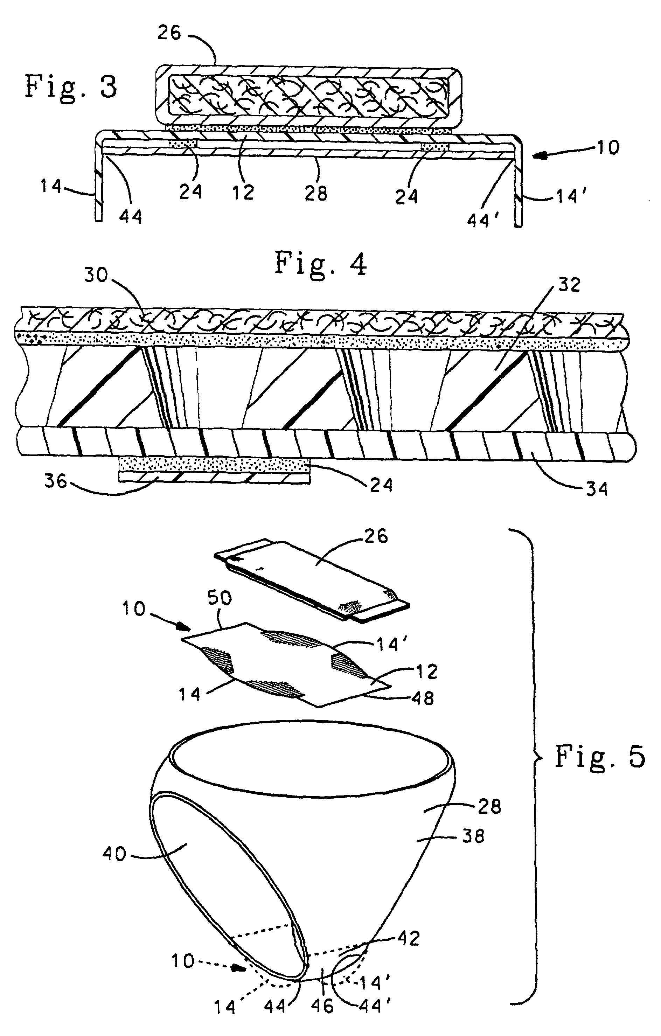

Figure 3 is a transverse cross-sectional view of the protective device 10 of the present invention in place between an absorbent article 26 and an undergarment 28. The absorbent article 26 can be any one of a myriad of such devices, and may be a feminine napkin or pad, or it may be a panty liner or shield. The protective device 10 of the present invention includes fastening adhesive strips 24. The fastening adhesive strips 24 provides an adhesive attachment means for securing the protective device 10 in the crotch portion of the undergarment 28. Any adhesive or glue used in the art for such purpose can be used herein, with pressure sensitive adhesives being preferred. Suitable adhesives are Century A-305-IV manufactured by the Century Adhesives corporation and Instant Lok 34-2823 manufactured by National Starch Company. As illustrated, a pair of fastening adhesive strips 24 are shown and are only of a width that is less than that of the elongated body 12 of the protective device 10. To employ the protective device 10, the user would first remove a release paper (shown below in Figure 4) and apply the protective device 10 with a slight pressure to the crotch area of the undergarment.

The absorbent article 26 similarly has an adhesive material so that it may be attached conventionally to an undergarment. However, in the present case, once the protective device 10 is in place in the undergarment, the user would remove release paper from the adhesive of the absorbent article 26 and then place the absorbent article 26 on top of the protective device 10 as illustrated in Figure 3.

As shown in Figure 3, the side extensions 14, 14' are positioned in their downfolded state. This represents one position that the side extensions 14, 14' may assume after the user's legs are inserted through the leg holes of the undergarment 28, and cause the side extensions 14, 14' to fold downward along the upper-inner thigh of the user.

Figure 4 is a transverse cross-sectional view of the protective device 10 of the present invention illustrating its multi-layered construction. The protective device 10 preferably includes a liquid permeable attachment surface or topsheet 30, an intermediate layer 32, and a liquid impermeable backsheet 34.

The liquid permeable topsheet 30 is not completely eclipsed by the absorbent article 26 as the side extensions 14, 14' extend beyond the sides of the absorbent article 26. Accordingly, the topsheet 30 is preferably composed of a compliant, soft-feeling material that is non-irritating to the parts of the user's skin with which it is in contact. The topsheet 30 can be made from any of the materials conventional for this type of use. Non-limiting examples of suitable materials that can be used as the topsheet 30 are non-woven polyester, polyethylene, polypropylene, nylon, and rayon and formed thermoplastic films. The preferred type of material is a spunbonded one that is pervious to liquids but is nevertheless non-absorbent. The particular material is selected so that the surface of the topsheet 30 remains dry and is thus more comfortable to the wearer. The recommended thickness of the topsheet 30 is between 1 and 2 mils with the preferred thickness being about 1 mil.

In the preferred embodiment of the present invention, the inner surface of the topsheet 30 is in contact with the intermediate layer 32. This contacting relationship results in liquid penetrating the topsheet 30 faster than if it were not in contact with the intermediate layer 32. The topsheet 30 can be maintained in contact with the intermediate layer 32 by applying adhesive, preferably in spaced, limited areas, to the inner surface of the topsheet 30. Examples of suitable adhesives used for this purpose include the acrylic emulsion E-1833BT manufactured by Rohm and Haas Company of Philadelphia, Pennsylvania and the acrylic emulsive WB3805 manufactured by H.B. Fuller Company of St. Paul, Minnesota. The adhesives can be applied by any of the common techniques well-known to those skilled in the art. For example, the adhesive can be applied by spraying, by padding, or by the use of transfer rolls.

The intermediate layer 32 is a soft barrier film and provides the protective device 10 with bulk and loft. The intermediate layer 32 is preferably composed of a hydrophobic resilient plastic webbing and may be made from any of the materials conventional for this type of use. Suitable materials are described in U.S. Patent No. 4,342,314, issued to Radel and Thompson on August 3, 1982 and U.S. Patent No. 4,463,045, issued to Ahr, Louis, Mullane and Ouellette on July 31, 1984. The intermediate layer 32 demonstrates some absorbency and fluid retention characteristics by funneling moisture away from the topsheet 30.

The liquid impermeable backsheet 34 is impervious to liquids and thus prevents body fluids which may not be absorbed by the absorbent article 26 from soiling the clothing of the user. Any polymerized barrier film used in the art for such purposes can be utilized for forming the backsheet 34. Suitable materials are embossed or non-embossed polyethylene films and laminated tissue.

The topsheet 30 has two purposes. First, it provides a soft layer for contact with the user's skin, thus making the protective device 10 comfortable to wear. Second, it provides a releasable anchor for the absorbent article 26. The recommended non-woven material of the topsheet 30 performs both of these tasks well by providing the protective device 10 with a soft feel and by allowing the absorbent article 26 to be removed without unintentional removal of the protective device 10 from the undergarment. (For added release characteristics, the topsheet 30 may be treated with silicone according to known methods.) The preferred construction of the protective device 10 provides for adhesive step increments whereby the bond of the adhesive strips 24 to the undergarment 28 is stronger than the adhesive bond between the absorbent article 26 and the topsheet 30. Accordingly, when the article 26 is removed, the protective device 10 stays in place until and unless intentionally removed by the user. Because of its liquid impermeable character, the backsheet 34 functions to provide actual moisture protection. The bottom sheet 34 is a polyethylene film such as that offered by Clopay Corporation (Cincinnati, Ohio) under the designation P18-0401 and by Ethyl Corporation (Terre Haute, Indiana) under the designation XP-39385.

The preferred embodiment of the protective device 10 includes the intermediate layer 32 as described above. However, the protective device 10 could be constructed without the intermediate layer 32, in that the topsheet 30 provides a release platform for the article 26 while the backsheet 34 provides necessary moisture protection and a surface for placement of the adhesive strips 24.

The adhesive strips 24 are covered with a release paper 36 to keep the adhesive strips 24 from sticking to extraneous surfaces prior to use. Any conveniently available release paper commonly used for such purposes can be used herein. Non-limiting examples of suitable release papers are BL 30 MG-A Silox EI/0 and BL 30 MG-A Silox 4 P/O, both of which are manufactured by the Akrosit Corporation.

Figure 5 is a partly exploded perspective view of the protective device 10 of the present invention situated both above an undergarment 28 prior to being fitted therein and also in place (shown in broken lines) within the undergarment 28. The undergarment 28 is of the type commonly worn by many women and well known as a panty. It comprises a front section 38, a back section 40, and a crotch portion 42 which joins the front and back sections 38 and 40, respectively. The crotch portion 42 comprises two side edges 44, 44' and a center crotch portion 46.

With reference first to the solid-lined illustration of the protective device 10 shown being applied to the undergarment 28, the protective device 10 is shown in its substantially planar shape as it would after purchase by the consumer but prior to installation into the undergarment 28. The protective device 10 is utilized by removing the release paper 36 and thereafter placing it in the undergarment 28. The elongated body 12 of the protective device 10 is placed in the crotch portion 42 of the undergarment 28 with a first end 48 extending toward the front section 38 and a second end 50 toward the back section 40 of the undergarment 28. The liquid impermeable backsheet 34 is in contact with the inner surface of the center crotch portion 46 of the panty. The adhesive strips 24 maintain the elongated body 12 in position. The side extensions 14, 14' are left extended laterally until such time as the user puts the undergarment 28 on, at which time the side extensions 14, 14' "automatically" fold downward to conform to the natural and particular shape of the wearer, thus resulting in a very comfortable and personalized fit. This is illustrated in the broken line illustration of Figure 5 of the protective device 10 in place in the undergarment 28. Because of the characteristics of the extensible regions 20, 20', 22, 22' in combination with the preferred materials, the protective device 10 holds this personal shape even between changings of the absorbent article 26 and provides a mechanism for the protective device 10 to fix itself to the undergarment 28.

Numerous benefits are derived from the use of the protective device 10 of the present invention. The side extensions 14, 14' are wrapped around each edge 44, 44' of the crotch portion 42 of the panty. This encapsulation of the undergarment crotch assists in preventing the body fluid from coming into contact with the center crotch portion 46 of the undergarment 28. Thus, the center crotch portion 46 of the undergarment 28 will not be soiled.

A further benefit is derived from the fact that the user need only change the protective device 10 after the absorbent article 26 has been changed a number of times. The need to change the protective device 10 is dictated only by the extent to which it is soiled.

Additionally, the protective device 10 of the present invention provides the user with the ability to better adjust placement of the protective device 10 to meet her particular needs. For example, when the user is about to sleep, the position of the protective device 10 may be adjusted forward or rearward (depending on whether or not the user sleeps on her stomach or back) to protect the undergarment from any fluid that might travel in these directions while keeping the absorbent article independently positioned in its most advantageous position. Known "winged" absorbent articles do not allow for this freedom because of their unitized construction. By moving the "winged" article fore or aft, the user sacrifices maximum absorbency.

There are at least two other benefits derived from using the protective device 10 of the present invention. One is that the portions of the side extensions 14, 14' extending over the edges 44, 44' provide an excellent gasket-like seal against the body. The other is that the absorbent article 26 is maintained in excellent contiguous relationship to the body.

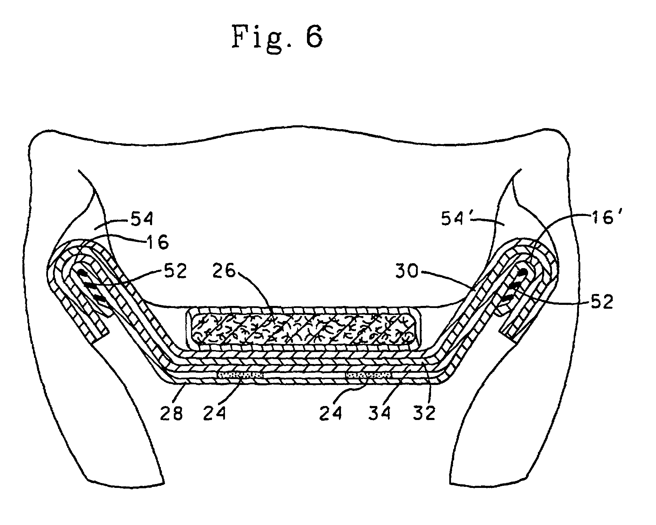

The edge of the crotch portion 42 of the undergarment 28 generally contains an elastic material 52, as illustrated in Figure 6, which is a fragmentary coronal view showing the sectional protective device 10, the absorbent article 26, and the undergarment 28 of Figure 5 place on a user. (A coronal view is the frontal plane that passes through the long axis of the body.) When the undergarment 28 is worn, the elastic material 52 of the crotch portion 42 generates an upward force, that is, against the body, due to the energy in the elastic and the fit of the undergarment 28. The approximate placement of the lines of juncture 16, 16' between the body and the edges 44, 44' of the crotch portion 42 of the undergarment 28 results in the upward force generated by the edge of the crotch portion of the undergarment 28 pushing the lines of juncture 16, 16' snugly into the groins 54, 54' of the body. This results in a gasket-like seal being formed along the lines of juncture 16, 16' between the side extensions 14, 14' and the body, thus maintaining body fluid in the crotch area and redirecting any wandering fluid back to the absorbent article 26 for absorption.

Another benefit derived from the lines of juncture 16, 16' being pushed snugly against the body is that the absorbent article 26 is forced into close proximity to and into conformity with the body, as is also illustrated in Figure 6. The maintenance of the article 26 against the body is known as "good body contact". Good body contact is beneficial because it provides a device to limit lateral flow of body fluid, Without such a device, body fluid would tend to flow quickly along the topsheet 30, thereby providing less time for it to be absorbed by the article 26.

Those skilled in the art can now appreciate from the foregoing description that the broad teachings of the present invention can be implemented in a variety of forms. Therefore, while this invention has been described in connection with particular examples thereof, the true scope of the invention should not be so limited since other modifications will become apparent to the skilled practitioner upon a study of the drawings, specification and following claims.

## Claims

1. A protective device for placement between an article (26) for absorbing body fluids and an undergarment (28), said undergarment (28) having an inner side, the protective device comprising an article attachment surface (30) for adhesive attachment of said article (26) for absorbing body fluids therein, an undergarment contacting surface (34) disposed adjacent said article attachment surface (30), and an area of adhesive (24) formed on said undergarment contacting surface (34), such that said undergarment contacting surface (34) is attachable to said inner side of said undergarment (28), **characterized in that** said device comprises:
a plurality of extensible regions (20, 20', 22, 22') for allowing expansion, said extensible regions (20, 20', 22, 22') being defined by corrugations of said article attachment surface (30) and said undergarment contacting surface (34), wherein
- said extensible regions (20, 20', 22, 22') are effectively free of said area of adhesive (24), and
- the bond of said area of adhesive (24) to said undergarment (28) is stronger than the adhesive bond between said absorbent article (26) and said article attachment surface (30).

2. The protective device according to Claim 1, **characterized in that** said corrugations comprise parallel and alternating ridges and grooves.

3. The protective device according to Claim 2, **characterized in that** the device includes a long axis and said parallel and alternating ridges and grooves are disposed transverse said long axis of said elongated body (12).

4. The protective device according to any one of Claims 1 to 3, **characterized in that** the device further includes an intermediate layer (32) formed between said article attachment surface (30) and said undergarment contacting surface (34), said extensible regions (20, 20', 22, 22') being further defined by said corrugations of said intermediate layer (32).

5. The protective device according to any of Claims 1 to 4, **characterized in that** said protective device comprises an elongated body (12) and a pair of side extensions (14, 14') extending laterally outwardly from said elongated body (12), each pair of said pair of side extensions (14, 14') being connectively associated with said body (12), said extensible regions (20, 20', 22, 22') being disposed intermediate said elongated body (12) and each of said pair of side extensions (14, 14').

6. The protective device according to Claim 5, **characterized in that** said side extensions (14, 14') extend laterally outwardly from said elongated body when the user nests said device into said crotch area (42) of said undergarment (28) and are folded when said undergarment (28) is put on by the wearer, said extensible regions (20, 20', 22, 22') expanding thereby to assume and hold an expanded position to conform to the crotch of the user.

## Patentansprüche

1. Schutzvorrichtung zur Anordnung zwischen einem Artikel (26) zum Absorbieren von Körperfluiden und einer Unterwäsche (28), wobei die Unterwäsche (28) eine Innenseite hat, wobei die Schutzvornchtung aufweist eine artikelseitige Anbringungsoberfläche (30) zur haftenden Anbringung des Artikels (26) zum Absorbieren von Körperfluiden, eine die Unterwäsche berührende Oberfläche (34), die angrenzend an die artikelseitige Anbringungsoberfläche (30) angeordnet ist, und eine Haftmittelfläche (24), die auf der die Unterwäsche berührenden Oberfläche (34) ausgebildet ist, derart, daß die die Unterwäsche berührende Oberfläche (34) an der Innenseite der Unterwäsche (28) anbringbar ist, **dadurch gekennzeichnet, daß** die Vorrichtung aufweist:
eine Mehrzahl von streckbaren Regionen (20, 20', 22, 22') zum Gestatten einer Erweiterung, wobei die streckbaren Regionen (20, 20', 22, 22') durch Riffelungen der artikelseitigen Anbringungsoberfläche (30) und der die Unterwäsche berührenden Oberfläche (34) begrenzt sind, wobei
- die streckbaren Regionen (20, 20' 22, 22') wirkungsfrei von der Haftmittelfläche (24) sind, und
- die Bindung der Haftmittelfläche (24) an der Unterwäsche (28) stärker ist, als die Haftmittelbindung zwischen dem absorbierenden Artikel (26) und der artikelseitigen Anbringungsoberfläche (30).

2. Schutzvorrichtung nach Anspruch 1, **dadurch gekennzeichnet, daß** die Riffelungen parallele und abwechselnde Rippen und Rillen umfassen.

3. Schutzvorrichtung nach Anspruch 2, **dadurch gekennzeichnet, daß** die Vorrichtung eine lange Achse umfaßt und die parallelen und abwechselnden Rippen und Rillen quer zu der langen Achse des länglichen Körpers (12) angeordnet sind.

4. Schutzvorrichtung nach einem der Ansprüche 1 bis 3, **dadurch gekennzeichnet, daß** die Vorrichtung ferner eine Zwischenlage (32) umfaßt, die zwischen der artikelseitigen Anbringungsoberfläche (30) und der die Unterwäsche berührenden Oberfläche (34) ausgebildet ist, wobei die streckbaren Regionen (20, 20', 22, 22') ferner durch die Riffelungen der Zwischenlage (32) begrenzt sind.

5. Schutzvorrichtung nach einem der Ansprüche 1 bis 4, **dadurch gekennzeichnet, daß** die Schutzvorrichtung einen länglichen Körper (12) und ein Paar Seitenerstrekkungen (14, 14') aufweist, die sich von dem länglichen Körper (12) seitlich nach außen erweitern, wobei jedes Paar des Paares von Seitenerstreckungen (14, 14') dem Körper (12) verbindend zugeordnet ist, wobei die streckbaren Regionen (20, 20', 22, 22') zwischen dem länglichen Körper (12) und jeder der Seitenerstreckungen (14, 14') des Paares angeordnet sind.

6. Schutzvorrichtung nach Anspruch 5, **dadurch gekennzeichnet, daß** sich die Seitenerstreckungen (14, 14') von dem länglichen Körper seitlich nach außen erweitern, wenn der Benutzer die Vorrichtung in den Schrittbereich (42) der Unterwäsche (28) einlegt, und gefaltet sind, wenn die Unterwäsche (28) von dem Träger angezogen ist, wobei sich die streckbaren Regionen (20, 20', 22, 22') dadurch erweitern, um eine erweiterte Stellung anzunehmen und zu halten, um sich an den Schritt des Benutzers anzupassen.

## Revendications

1. Dispositif de protection conçu pour être placé entre un article (26) destiné à absorber les fluides corporels, et un sous-vêtement (28), ledit sous-vêtement (28) ayant un côté intérieur, le dispositif de protection comprenant une surface de fixation d'article (30) en vue d'une fixation adhésive dudit article (26) dans lequel sont absorbés les fluides corporels, une surface de contact de sous-vêtement (34) placée à proximité immédiate de ladite surface de fixation d'article (30), et une zone d'adhésif (24) formée sur ladite surface de contact de sous-vêtement (34) afin que ladite surface de contact de sous-vêtement (34) puisse être fixée audit côté intérieur dudit sous-vêtement (28), **caractérisé en ce que** ledit dispositif comprend :
une pluralité de régions extensibles (20, 20', 22, 22') destinées à permettre une extension, lesdites régions extensibles (20, 20', 22, 22') étant définies par des ondulations de ladite surface de fixation d'article (30) et de ladite surface de contact de sous-vêtement (34), dans lequel
- lesdites régions extensibles (20, 20', 22, 22') sont efficacement exemptes de ladite zone d'adhésif (24), et
- la liaison entre ladite zone d'adhésif (24) et ledit sous-vêtement (28) est plus solide que la liaison adhésive entre ledit article absorbant (26) et ladite surface de fixation d'article (30).

2. Dispositif de protection selon la revendication 1, **caractérisé en ce que** lesdites ondulations comprennent des arêtes et des rainures parallèles et alternées.

3. Dispositif de protection selon la revendication 2, **caractérisé en ce que** le dispositif comprend un axe long, et lesdites arêtes et rainures parallèles et alternées sont placées transversalement audit axe long dudit corps allongé (12).

4. Dispositif de protection selon l'une quelconque des revendications 1 à 3, **caractérisé en ce que** le dispositif comprend, en outre, une couche intermédiaire (32) formée entre ladite surface de fixation d'article (30) et ladite surface de contact de sous-vêtement (34), lesdites régions extensibles (20, 20', 22, 22') étant définies, en outre, par lesdites ondulations de ladite couche intermédiaire (32).

5. Dispositif de protection sclon l'une quelconque des revendications 1 à 4, **caractérisé en ce que** ledit dispositif de protection comprend un corps allongé (12) et une paire de prolongements latéraux (14, 14') s'étendant latéralement vers l'extérieur à partir dudit corps allongé (12), chaque paire de ladite paire de prolongements latéraux (14, 14') étant associée par liaison avec ledit corps (12), lesdites régions extensibles (20, 20', 22, 22') étant placées entre ledit corps allongé (12) et chacun de ladite paire de prolongements latéraux (14, 14').

6. Dispositif de protection selon la revendication 5, **caractérisé en ce que** lesdits prolongements latéraux (14, 14') s'étendent latéralement vers l'extérieur à partir dudit corps allongé lorsque l'utilisateur introduit ledit dispositif dans ladite zone d'entrejambe (42) dudit sous-vêtement (28), et sont pliés lorsque ledit sous-vêtement (28) est mis par le porteur, lesdites régions extensibles (20, 20', 22, 22') s'étendant de façon à prendre et à maintenir, ainsi, une position d'extension afin d'épouser l'entrejambe de l'utilisateur.
